# EUROPEAN PATENT APPLICATION

(11) **EP 1 587 043 A2**
(43) Date of publication of application: **19.10.2005**
(21) Application number: 05006810.5
(22) Date of filing: 29.03.2005
(51) Int. Cl.: G08C 19/00

(54) **Communication device and communication cable**

(30) Priority: 08.04.2004 JP 2004114720
(71) Applicant: Olympus Corporation, Shibuya-ku, Tokyo (JP)
(72) Inventor: Sekiguchi, Kiyoshi, Hachioji-shi, Tokyo (JP)
(74) Representative: von Hellfeld, Axel

(57) **Abstract**

When communication is to be carried out between a central control unit and an appliance that uses a different form of communication from the central control unit, a converting part for converting commands is provided in a communication path (communication device which may be a communication cable) between the central control unit and the appliance. By this means, communication between the central control unit and peripheral appliances is made possible and central control of the peripheral appliances is possible notwithstanding that their respective forms of communication are different. Therefore, the number of appliances that can be connected to the central control unit is increased. Preferably, the converting part can be set to a mode that corresponds to a respective appliance among a plurality of converting modes. An electronic circuit corresponding to the converting part is for example provided in a communication cable connecting the central control unit to an appliance.

## Description

### CROSS-REFERENCE TO RELATED APPLICATION

This application is based upon and claims the benefit of priority from prior Japanese Patent Application No. 2004-114720, filed on April 8, 2004.

### BACKGROUND OF THE INVENTION

### 1. Field of the Invention

This invention relates to a communication device for effecting communication between a central control unit and an object appliance.

### 2. Description of the Related Art

In recent years, with developments in medical treatment technology, and in particular medical treatment appliances have become rich in variety and numerous in function. Such medical treatment appliances include electric scalpel instruments, ultrasonic suction devices, laser scalpel instruments and others. Although these medical treatment appliances are sometimes used on their own, they are also often used as part of a larger medical treatment system. Using these medical treatment appliances, a surgeon holds a surgical implement such as an electric scalpel in a hand to treat a lesion of an afflicted area or the like.

This kind of medical treatment system has a central control unit capable of controlling the operations of multiple medical treatment appliances such as an electric scalpel device, a stomach-inflating device, and a camera device by means of remote control units such as foot switches, hand switches and remote controllers.

The central control unit selectively displays a desired medical treatment appliance, for example selected by operation of a remote control unit on a display part such as a monitor so that the respective medical treatment appliance can be easily recognized. Then, the central control unit centrally controls the medical device selectively displayed on the display part on the basis of user manipulations of the remote control units.

However, the forms of communication used by peripheral appliances such as anesthesia unit systems made up of electrocardiograms and anesthesia units, and beds and lights and so on, differ. Consequently, in related art, peripheral appliances such as anesthesia unit systems, beds and lights have been individually controlled. For example in the case of lights, brightness control and so on have been carried out by a nurse using a remote controller (not shown).

Here, generally, communication between different appliances has been carried out by protocol processing.

In related art, communication devices used for communication between appliances include for example those proposed in JP-UM-A-4-135044 and JP-A-8-51471.

In a communication device set forth in JP-UM-A-4-135044, all or part of a communication device for carrying out the above-mentioned protocol processing is provided inside a connector extending from a data processing unit.

A communication device set forth in JP-A-8-51471, on the other hand, is constructed to convert communication data input through a first interface with a predetermined interface agreement and output to a second interface.

However, these communication devices of related art only enable communication between appliances having different protocols, they fail to realize communications between appliances having different communication forms other than protocols.

Consequently, even when these communication devices are used, in the medical treatment systems discussed above, a central control unit for stomach-inflators and cameras and so on can not control peripheral appliances that use different forms of communication (e.g., the above-mentioned anesthesia unit system, bed, lights, etc.).

Here, in a medical treatment system of the kind discussed above, it would be costly to employ the same communication forms in all peripheral appliances to enable central control of electrocardiograms, anesthesia units, beds, lights and so on.

The present invention was made in view of these points, and it is an object of the invention to provide a communication device which makes communication between a central control unit and an appliance possible and thereby makes central control of the appliance possible, even if their forms of communication are different.

### BRIEF SUMMARY OF THE INVENTION

In the invention, in a communication path (for example a communication cable or a communication device) connecting a central control unit to an appliance, there is provided a converting part for converting a communication command from the central control unit into a communication command for the appliance. Here, a communication command is a transmitted command. If this kind of communication device or communication cable is employed, communication becomes possible between a central control unit and an appliance even if their forms of communication are different. In particular, peripheral appliances having different forms of communication can be connected to an existing central control unit, and the number of appliances that can be controlled by the central control unit increases.

An output switching part for switching the communication commands converted by the converting part in correspondence with the object appliance can be further provided. In this case, multiple appliances can be handled by one type of communication device or communication cable.

A communication device according to the invention can have a storing part for storing data relating communication commands to be converted by the converting part, and the converting part converts preset communication commands output from the central control unit in correspondence with the object appliance on the basis of the data relating to the communication commands stored in the storing part. In this case, data conversion details can be easily changed by a data change.

The converting part may carry out conversion between forms of communication using different numbers of signal lines. And, the converting part may carry out conversion between parallel communication and serial communication.

A communication device according to the invention has the effect of making communication between a central control unit and an appliance possible and thereby making central control of the appliance possible, even if their forms of communication are different.

### BRIEF DESCRIPTION OF THE DRAWINGS

These and other features, aspects and advantages of the apparatus and methods of the present invention will become better understood with regard to the following description, appended claims, and accompanying drawings, where:
Fig. 1 is an overall construction view of an endoscope surgery system having an embodiment of a communication device according to the invention;
Fig. 2 is a view showing the system controller, the communication cable and the peripheral appliance shown in Fig. 1;
Fig. 3 is an enlarged schematic view of the communication cable of Fig. 2;
Fig. 4 is a circuit block diagram showing the internal construction of the communication cable of Fig. 3;
Fig. 5A and Fig. 5B are views illustrating communication command conversion processing carried out by a communication cable between a system controller and an HDD recorder, Fig. 5A being a schematic view illustrating the communication command conversion processing between the system controller and the HDD recorder carried out by the communication cable and Fig. 5B a view illustrating a communication command conversion table used in the conversion processing of Fig. 5A;
Fig. 6A and Fig. 6B are views illustrating communication command conversion processing carried out by a communication cable between a system controller and a light, Fig. 6A being a schematic view illustrating the communication command conversion processing carried out between the system controller and the light by the communication cable, and Fig. 6B a view illustrating a communication command conversion table used in the conversion processing of Fig. 6A;
Fig. 7 is a flow chart showing start processing carried out by the control part in Fig. 4;
Fig. 8 is a flow chart showing a normal mode of start processing in Fig. 7; and
Fig. 9 is a flow chart showing a setting mode of Fig. 8.

### DETAILED DESCRIPTION OF THE PREFERRED EMBODIMENTS

An embodiment of the invention will be described below with reference to the accompanying drawings.

In this embodiment the invention is applied to an endoscope surgery system. However, those skilled in the art will appreciate that the endoscope surgery system is given by way of example only and not to limit the scope or spirit of the invention.

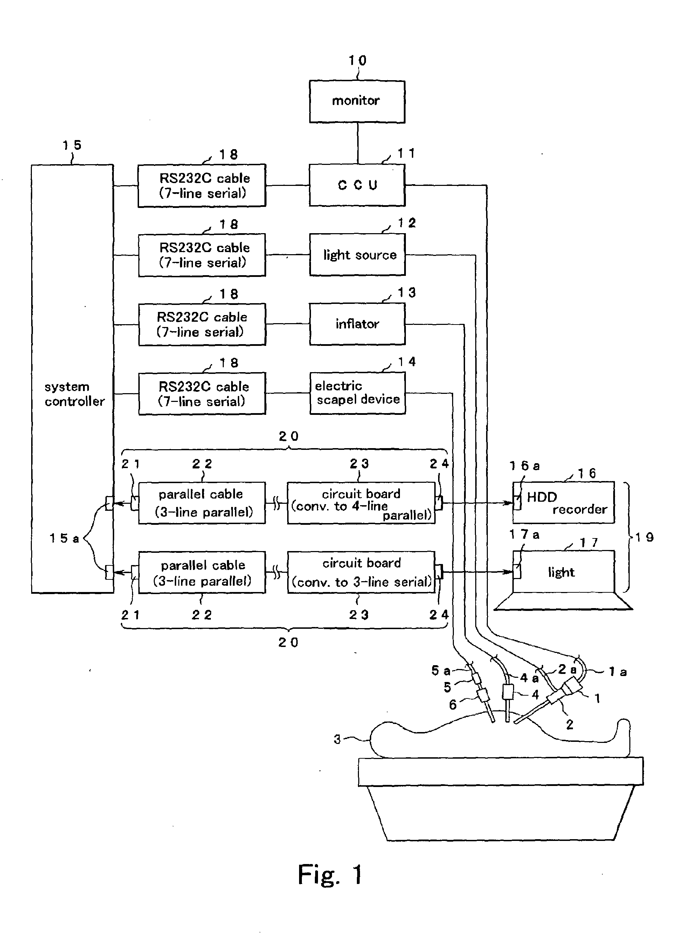

Fig. 1 through Fig. 6 pertain to such embodiment. As shown in Fig. 1, an endoscope surgery system has a rigid endoscope (hereinafter simply endoscope) 2 fitted with a TV camera 1 incorporating an image pick-up device. This endoscope 2 is inserted through a trocar (not shown) into the stomach cavity of a patient 3 lying on an operating table as is known in the art. In the endoscope surgery system a stomach-inflating trocar 4 for inflating the stomach and an operating appliance trocar 6 for guiding an electric scalpel 5, which is an operating appliance, into the stomach cavity, are also inserted into the patient 3.

Also in the endoscope surgery system, a signal cable 1a connected to the TV camera 1, a light guide cable 2a connected to the endoscope 2, a stomach-inflating tube 4a connected to the stomach-inflating trocar 4, and a signal cable 5a connected to the electric scalpel 5 are respectively connected to a CCU (Communication Control Unit) 11, a light source 12, an inflator 13, and an electric scalpel device 14.

The CCU 11 performs signal processing with respect to the image pick-up device built into the TV camera 1, and displays an endoscope image on a monitor 10. The light source 12 supplies illuminating light to the endoscope 2. The inflator 13 supplies air or other gas for stomach inflation to the stomach cavity of the patient 3 through the stomach-inflating trocar 4. The electric scalpel device 14 supplies highfrequency electric power for cauterization to the electric scalpel 5.

The CCU 11, the light source 12, the inflator 13 and the electric scalpel device 14 are centrally controlled by a system controller 15 serving as a central control unit. The endoscope surgery system also includes peripheral appliances 19 such as an HDD recorder 16 for recording the image signal output from the CCU 11 and a light 17 constituting a lighting device for illuminating the operating theater.

The system controller 15 is for example capable of 7-line serial and 3-line parallel communication only. Accordingly, it can control medical devices compatible with 7-line serial and 3-line parallel communication with ordinary communication cables as communication devices.

Medical treatment appliances such as the CCU 11, the light source 12, the stomach-inflator 13 and the electric scalpel 14 are capable of 7-line serial communication, and these are connected to the system controller 15 by RS232C cables 18 used for this form of communication and are controllable by common 7-line serial communication.

The HDD recorder 16 and the light 17 and other peripheral appliances 19 employ forms of communication different from the 7-line serial and 3-line parallel communication handled by the system controller 15. For example, the HDD recorder 16 employs 4-line parallel communication and the light 17 employs 3-line serial communication. Because of this, in related art, the HDD recorder 16 and the light 17 and other peripheral appliances 19 have been separately controlled. For example, static image capture with the HDD recorder 16 and brightness adjustment of the light 17 and so on have been carried out by a nurse using an independent remote controller (not shown).

In the embodiment of Figs. 1-6, peripheral appliances 19 having different forms of communication such as the HDD recorder 16 and the light 17 are connected to the system controller 15 and centrally controlled.

That is, the HDD recorder 16 and the light 17 are each connected to the system controller 15 by a communication cable 20 constituting a communication device.

The communication cable 20 is made up of a controller side connector 21 serving as an input part, a parallel cable 22, a circuit board 23, and a peripheral appliance side connector 24 serving as an output part.

The controller-side connector 21 is connected to a connector socket 15a provided on the system controller 15. The parallel cable 22 extends from the controller-side connector 21 and is electrically connected to the circuit board 23. This parallel cable 22 is a for example 3-line parallel communication cable, by which communication with the system controller 15 is possible.

The circuit board 23 carries a CPU for executing command conversion processing on communication commands from the system controller 15 in correspondence with a peripheral appliance. This circuit board 23 for example carries out command conversion processing in correspondence with a peripheral appliance to convert 3-line parallel communication to 4-line parallel communication or 3-line serial communication. The above-mentioned peripheral appliance side connector 24 is electrically connected to the circuit board 23. This peripheral appliance side connector 24 is connected to a connector socket 16a, 17a provided in the HDD recorder 16, light 17 or other peripheral appliance 19.

Next, a specific detailed construction of this communication cable 20 will be described.

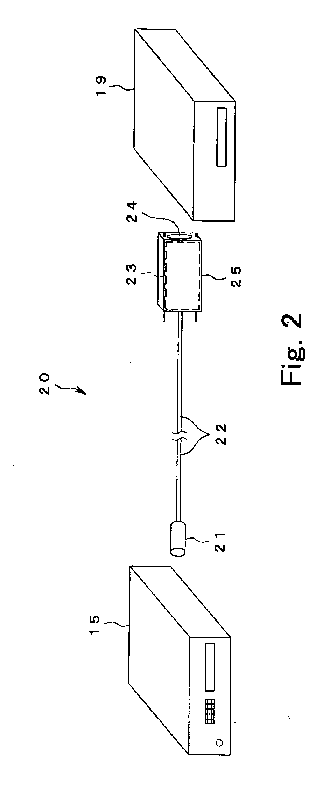

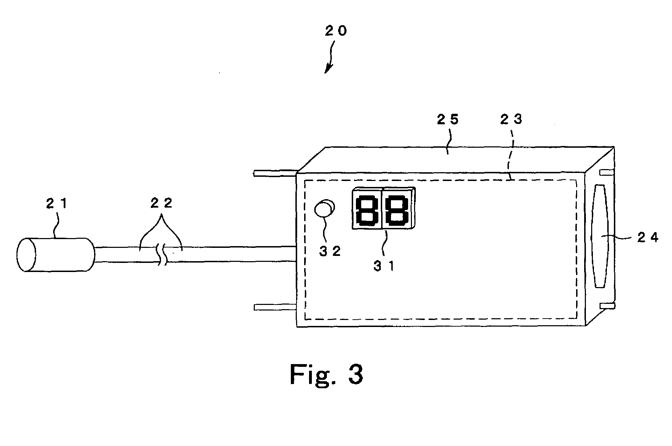

In the communication cable 20 shown in Fig. 2 and Fig. 3, the circuit board 23 is housed in a case 25, and the peripheral appliance side connector 24 projects from this case 25.

A display part 31 and a setting button 32 are provided on the case 25. The display part 31 displays the name of the peripheral appliance with respect to which communication command conversion processing is being carried out. The display part 31 shown in Fig. 3 is an LED display and displays the device name in digits, but alternatively it may be an LCD or some other display and may display the device name in letters.

The setting button 32 is a button for performing a setting operation for communication command conversion processing. When this setting button 32 is pressed steadily it goes into a setting mode, and a setting operation, which will be discussed later, becomes possible.

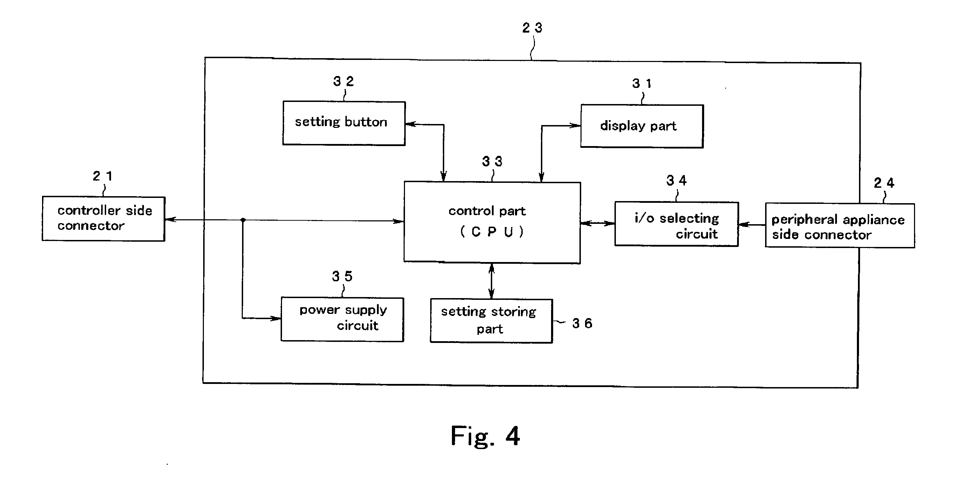

As shown in Fig. 4, the setting button 32 and the display part 31 are mounted on the circuit board 23. A control part 33 serving as a converting part, an input/output selecting circuit 34 serving as an output switching part, a power supply circuit 35, and a settings storing part 36 serving as a storing part are also mounted on this circuit board 23. The control part 33 includes for example a CPU (Central Processing Unit), and performs communication command conversion processing for converting communication commands in correspondence with the respective peripheral appliance.

The input/output selecting circuit 34 switches communication commands converted by the control part 33 to correspond with a respective peripheral appliance 19. This input/output selecting circuit 34 also selects whether a signal from the system controller 15 is output to the peripheral appliance 19 or a signal from the peripheral appliance 19 is input to the system controller 15.

The settings storing part 36 stores as conversion data for converting input communication commands for example as a command conversion table, which will be further discussed later. The settings storing part 36 is made up of electrically erasable (rewriteable) ROM such as for example EEPROM (Electrically Erasable Programmable Read-Only Memory). The power supply circuit 35 supplies a power originated from the system controller 15 to the other circuits.

Next, communication command conversion processing carried out by the control part 33 will be described.

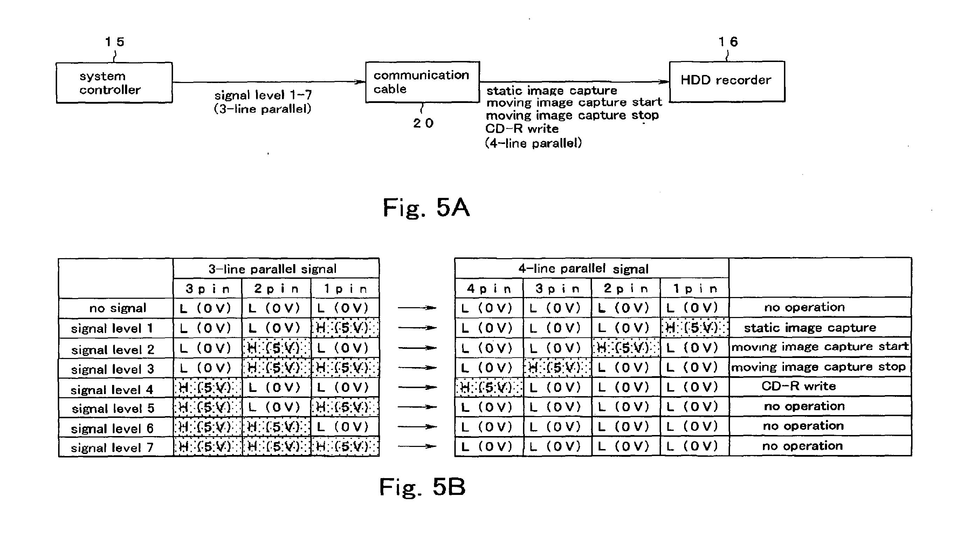

First, with reference to Figs. 5A and 5B, the case of the HDD recorder 16 being used as a peripheral appliance 19 will be discussed. As shown in Fig. 5A, the system controller 15 and the HDD recorder 16 are connected by the communication cable 20. The controller-side connector 21 uses for communication the terminals of 3 pins among the 7 pins. The peripheral appliance side connector 24, on the other hand, uses for communication the terminals of 4 pins among the 7 pins. The controller-side connector 21 uses the terminal of 1 pin among the 7 pins for power supply.

The system controller 15 has signal levels 1 to 7 of a 3-line parallel signal as communication commands. The communication cable 20 converts these signal levels 1 to 7 to signal levels 1 to 7 of a 4-line parallel signal as corresponding communication commands for the HDD recorder 16. The communication commands for the HDD recorder 16 may include for example commands for static image capture, moving image capture start, moving image capture stop, and CD-R write.

The signal levels 1 to 7 of the 3-line parallel signal output from the system controller 15 and the signal levels 1 to 7 of the 4-line parallel signal into which these are converted are for example set as detailed in a command conversion table for the HDD recorder 16 shown in Fig. 5B.

Here, before the system controller 15 operates, the 3-line parallel signal pins 1 to 3 continue in a no-signal state. When the system controller 15 operates, the 3-line parallel signal pins 1 to 3 assume one of the above-mentioned signal level 1 to 7 states, and return to the no-signal state after 500ms elapses.

When it is not receiving a 3-line parallel signal level 1 to 7, the communication cable 20 keeps the 4-line parallel signal pins 1 to 4 for the HDD recorder in a no-operation state. When it receives a 3-line parallel signal level 1 to 7 from the system controller 15, the communication cable 20 outputs a corresponding converted communication command such as static image capture, moving image capture start, moving image capture stop, or CD-R write, as shown in Fig. 5B, to the 4-line parallel signal pins 1 to 4 for the HDD recorder, for 100ms, and after 100ms elapses returns them to the no-operation state.

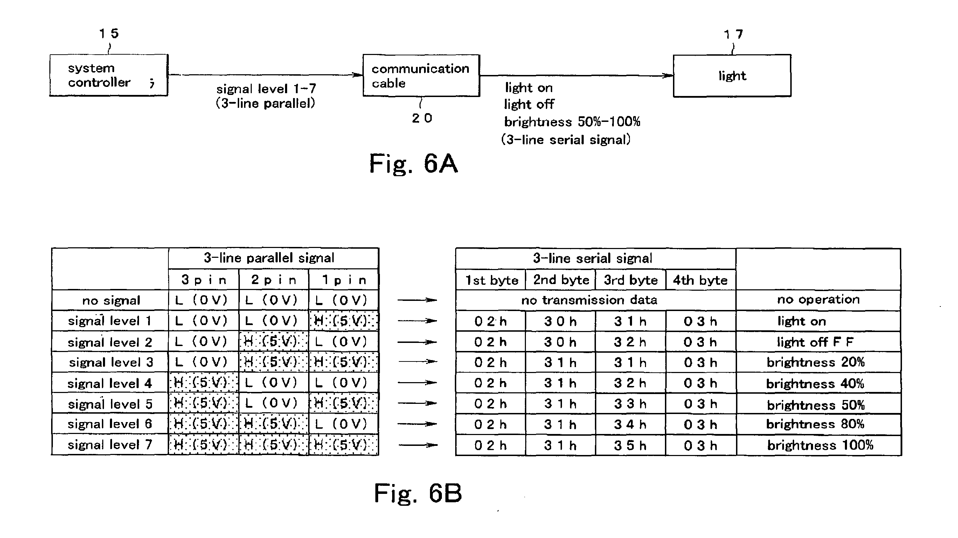

Next, with reference to Figs. 6A and 6B, the case of the light 17 being used as a peripheral appliance 19 will be discussed. As shown in Fig. 6A, the system controller 15 and the light 17 are connected by a communication cable 20.

The controller-side connector 21 uses for communication the terminals of 3 pins among the 7 pins, and the peripheral appliance side connector 24 uses for communication the terminals of 3 pins among the 7 pins. The controller-side connector 21 also uses the terminal of 1 pin among the 7 pins for power supply.

The system controller 15 has signal levels 1 to 7 of a 3-line parallel signal as communication commands. The communication cable 20 converts these signal levels 1 to 7 to corresponding 3-line serial signal levels 1 to 7 and outputs them as communication commands for the light 17. The communication commands for the light 17 are for example commands such as light on, light off, and brightness 50% to 100%.

The signal levels 1 to 7 of the 3-line parallel signal output from the system controller 15 and the signal levels 1 to 7 of the 3-line serial signal to which these signal levels 1 to 7 are converted are for example set as detailed in the command conversion table for the light 17 shown in Fig. 6B.

Here, before the system controller 15 operates, the 3-line parallel signal pins 1 to 3 continue in a no-signal state. When the system controller 15 operates, the 3-line parallel signal pins 1 to 3 assume one of the above-mentioned signal level 1 to 7 states, and return to the no-signal state after 500ms elapses.

When it is not receiving a 3-line parallel signal level 1 to 7, the communication cable 20 does not perform serial command transmission for the light 17, and continues a no-operation state. When it receives a 3-line parallel signal level 1 to 7 from the system controller 15, the communication cable 20 outputs a corresponding converted communication command such as light on, light off, brightness 50% to 100%, as shown in Fig. 6B, to the 3-line serial signal pins 1 to 3 for the light, for 100ms, and after 100ms elapses returns them to the no-operation state.

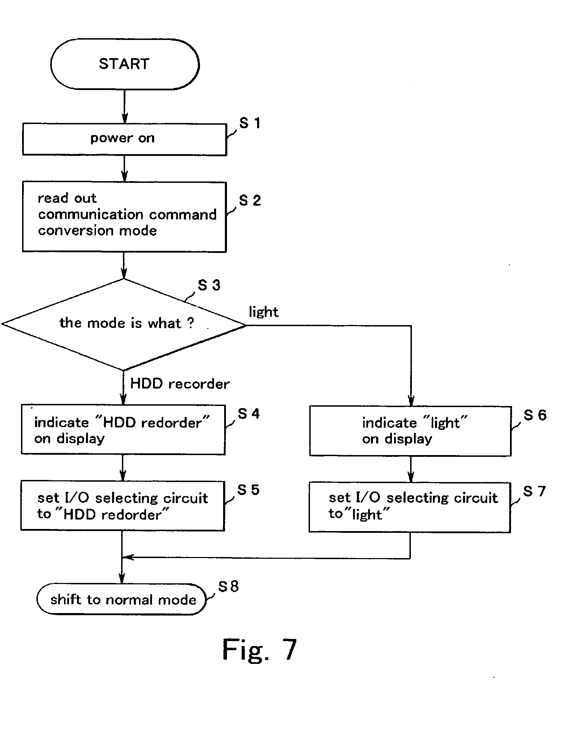

The communication cable 20 constructed like this operates for example in accordance with the flow charts shown in Fig. 7 through Fig. 9. A user connects the system controller 15 and the respective peripheral appliance 19 with a communication cable 20. Here, the cases of an HDD recorder 16 and a light 17 being used as peripheral appliances 19, as discussed above, will be described.

First, a start processing flow chart will be explained.

As shown in Fig. 7, first, an operator turns on the power supply of the system controller 15 (step S1). The communication cable 20 is supplied with power from the system controller 15 through 1 pin of the controller-side connector 21.

The control part 33 of the communication cable 20 reads out a communication command conversion mode from the settings storing part 36 (step S2). The control part 33 determines what the communication command conversion mode read out is (step S3).

When the communication command conversion mode read out is an HDD Recorder mode, the control part 33 indicates the HDD recorder 16 on the display part 31 (step S4), and sets the input/output selecting circuit 34 to the HDD recorder 16 (step S5). The control part 33 then shifts to a Normal mode (step S8).

On the other hand, when the communication command conversion mode read out is a Light mode, the control part 33 indicates the light 17 on the display part 31 (step S6), and sets the input/output selecting circuit 34 to the light 17 (step S7). The control part 33 then shifts to the Normal mode (step S8).

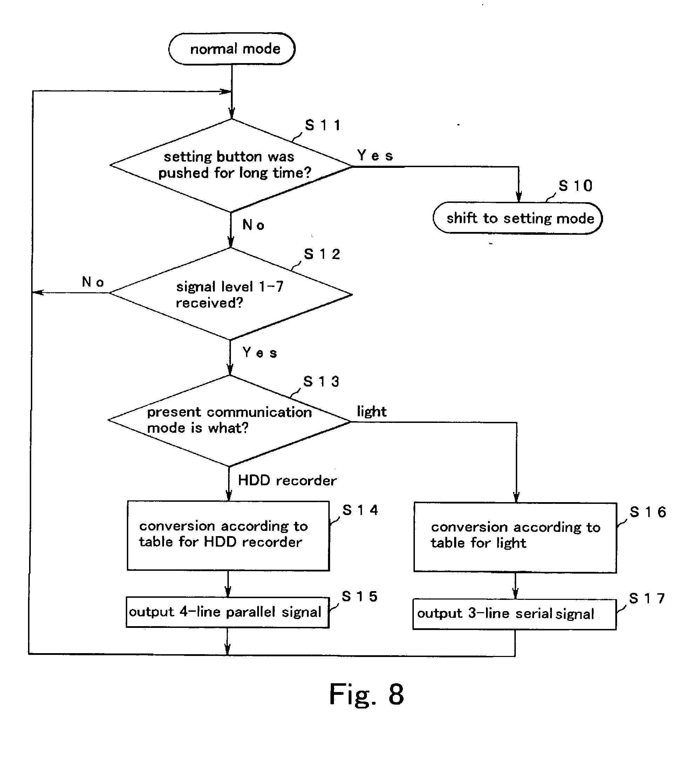

As shown in Fig. 8, in the Normal mode, the control part 33 determines whether or not the setting button 32 has been steadily pushed by a user for a predetermined time (step S11).

When the setting button 32 has been steadily pushed, the control part 33 shifts to a Setting mode (step S10), which will be further discussed later. When on the other hand the setting button 32 has not been pressed steadily, the control part 33 continues in the Normal mode.

The control part 33 determines whether or not any one of the signal levels 1 to 7 of the 3-line parallel signal output from the system controller 15 has been received (step S12).

When one of the signal levels 1 to 7 is not received from the system controller 15, the method loops back to step S11 to determine if the setting button is steadily pushed for a predetermined time. When one of the signal levels 1 to 7 has been received from the system controller 15, the control part 33 determines what the present communication command conversion mode is set to (step S13).

When the current communication command conversion mode is the HDD Recorder mode, the control part 33 converts the signal from the system controller 15 into a communication command in accordance with the communication command conversion table for the HDD recorder stored in the settings storing part 36 (step S14).

The control part 33 then outputs the signal obtained by communication command conversion as a 4-line parallel signal (step S15).

Here, if the 3-line parallel signal output from the system controller 15 is at signal level 2, or LH (0V, 0V, 5V), it is converted to LLLH (0V, 0V, 0V, 5V), which is the static image capture operation command. The converted signal is output to the HDD recorder 16 through the peripheral appliance side connector 24. The HDD recorder 16 receiving this signal performs a static image capture operation and records static image data.

When on the other hand the present communication command conversion mode is the Light mode, the control part 33 converts the signal from the system controller 15 into the communication command in accordance with the command conversion table for the light stored in the settings storing part 36 (step S16).

The control part 33 then outputs the signal obtained by communication command conversion as a 3-line serial signal (step S17).

Here, if the 3-line parallel signal output from the system controller 15 is at signal level 2, or LLH (0V, 0V, 5V), it is converted to first byte = 02h, second byte = 30h, third byte = 31h, and fourth byte = 03h as the 3-line serial signal that is the ' light on' command. The converted signal is output to the light 17 through the peripheral appliance side connector 24. The light 17 receiving the signal turns on and illuminates the operating theater.

By this means the communication cable 20 can execute communication command conversion processing on a signal output from the system controller 15 and output it to the HDD recorder 16 or the light 17, and these peripheral appliances 19 having different forms of communication can be controlled.

Next, the Setting mode will be explained.

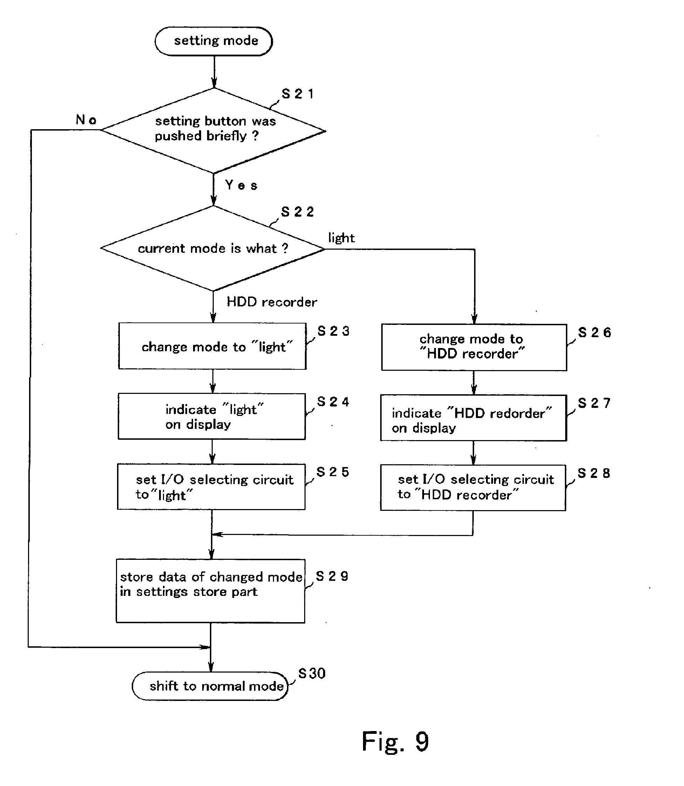

As shown in Fig. 9, in the Setting mode, the control part 33 determines whether or not a communication command conversion mode to be set has been selected by a user pushing the setting button 32 briefly (step S21). Where it is determined that the setting button 32 is not pushed briefly, the method proceeds to step S30 and shifts to Normal mode.

Where it is determined that the setting button 32 is pushed briefly, communication command conversion modes to be set are displayed on the display part 31 and an operator can select one after the other in a toggle fashion.

When a communication command conversion mode to be set has been selected, the control part 33 determines what the current communication command conversion mode is (step S22).

When the current communication command conversion mode is the HDD Recorder mode, the control part 33 changes the communication command conversion mode to the light 17 (step S23). The control part 33 changes the display on the display part 31 to indicate the light 17 (step S24) and changes the input/output selecting circuit 34 over to serve the light 17 (step S25).

When on the other hand the current communication command conversion mode is the Light mode, the control part 33 changes the communication command conversion mode to the HDD recorder 16 (step S26). The control part 33 changes the display on the display part 31 to indicate the HDD recorder 16 (step S27) and changes the input/output selecting circuit 34 over to serve the HDD recorder 16 (step S28).

The control part 33 stores data of the changed communication command conversion mode in the settings storing part 36 (step S29) and shifts to Normal mode at step S30.

By this means, the communication cable 20 can change the set communication command conversion mode in correspondence with the peripheral appliance 19, and data of the changed setting can be stored in the settings storing part 36.

In this embodiment, an HDD recorder 16 and a light 17 were used as examples of peripheral appliances 19 having different forms of communication. However, other peripheral appliances 19 are possible, such as an electrocardiogram, an anesthesia system having an anesthesia unit, and/or equipment such as a bed may be connected to the system controller 15 and centrally controlled.

And, although in the foregoing embodiment communication between the system controller 15 and the peripheral appliances 19 was carried out by a communication cable 20 being connected as a communication device between them, the invention is not limited to this, and as long as there is provided a control part that performs communication command conversion processing as a converting part, the input part or the output part may alternatively be constructed so that communication can be carried out for example wirelessly in which the communication device can comprise at least one transmitter and receiver for at least partial wireless communication between the peripheral appliances 19 and the system controller 15.

As a result, the communication cable 20 of this embodiment can be connected to a system controller 15 (central control unit) and central control carried out even if forms of communication differ.

Although in the embodiment described above the invention was applied to an endoscope surgery system, the invention is not limited to this and can of course be applied to industrial and other systems for centrally controlling object devices.

Furthermore, embodiments constructed by combining the foregoing embodiment partially also belong to the invention.

The communication device of this invention, by making possible communication between a central control unit and peripheral appliances even when their forms of communication differ and thereby making central control of the peripheral appliances possible, is useful wherever object appliances are to be centrally controlled in the medical treatment field, industrial fields and other fields.

While there has been shown and described what are considered to be preferred embodiments of the invention, it will, of course, be understood that various modifications and changes in form or detail could readily be made without departing from the spirit of the invention. It is therefore intended that the invention be not limited to the exact forms described and illustrated, but should be constructed to cover all modifications that may fall within the scope of the appended claims.

## Claims

1. A communication device (20) comprising an input part for inputting a preset communication command output from a central control unit (15),
**characterized in that** the communication device further comprising:
a converting part (23, 33) for converting the communication command input from the input part in correspondence with an object appliance; and
an output part (24) for outputting a communication command converted by the converting part to the object appliance (19).

2. A communication device according to claim 1, further comprising an output switching part for switching the communication command to be converted by the converting part in correspondence with the object appliance.

3. A communication device according to claim 1, further comprising a storing part for storing data relating to the communication command to be converted by the converting part, wherein the converting part converts the communication command output from the central control unit in correspondence with the object appliance on the basis of the data relating to the communication command stored in the storing part.

4. A communication device according to claim 3, wherein the converting part can change the data relating to the communication command stored in the storing part.

5. A communication device according to claim 1, wherein the converting part has a CPU and the CPU has a first operating mode in which the CPU converts communication commands input from the input part in a predetermined converting mode and a second operating mode in which the CPU switches converting modes.

6. A communication device according to claim 1, wherein the converting part carries out conversion between forms of communication requiring different numbers of signal lines.

7. A communication device according to claim 1, wherein the converting part carries out conversion between parallel communication and serial communication.

8. A communication device according to claim 1, wherein at least one of the input part, converting part and output part is disposed in a cable operatively connected between the central control unit and the object appliance.

9. A communication cable (20), comprising an input part (21) for inputting a preset communication command output from a central control unit (15),
**characterized in that** the communication cable further comprising:
a converting part (23, 33) for converting the communication command input by the input part (21) in correspondence with an object appliance; and
an output part (24) for outputting the communication command converted by the converting part to the object appliance (19).

10. A communication cable according to claim 9, further comprising an output switching part for switching the communication command converted by the converting part in correspondence with the object appliance.

11. A communication cable according to claim 9, further comprising a storing part for storing data relating to the communication command to be converted by the converting part, wherein the converting part converts the communication command output from the central control unit in correspondence with the object appliance on the basis of the data relating to the communication command stored in the storing part.

12. A communication cable according to claim 11, wherein the converting part can change the data relating to the communication command stored in the storing part.

13. A communication cable according to claim 9, wherein the converting part has a CPU and the CPU has a first operating mode in which the CPU converts communication commands input from the input part in a predetermined converting mode and a second operating mode in which the CPU switches converting modes.

14. A method for transmitting a command from a central control (15) unit to an object appliance controlled by a form of communication different from that of the central control unit,
**characterized in that** the method comprising:
connecting the central control unit and the object appliance with a communication device (20);
setting a communication command converting circuit (23, 33) provided in the communication device (20) to a setting at which it serves the object appliance; outputting a predetermined command from the central control unit (15) to the object appliance (19);
converting the command in the communication command converting circuit in correspondence with the setting; and
outputting the converted command to the object appliance (19).

15. The method according to claim 14, wherein the communication device is a communication cable.
